# EUROPEAN PATENT APPLICATION

(11) **EP 4 306 038 A1**
(43) Date of publication of application: **17.01.2024**
(21) Application number: 22184105.9
(22) Date of filing: 11.07.2022
(51) Int. Cl.: A61B 3/113

(54) **SYSTEM FOR TRACKING EYE PARAMETERS DURING SLEEP**

(71) Applicant: UNIVERSITE DE GENEVE, 1211 Geneva 4 (CH)
(72) Inventor: YUZGEC, Ozge, 1205 Geneva - Plainpalais (CH); HUBER, Daniel, 1213 Geneva - Petit-Lancy (CH)
(74) Representative: reuteler & cie SA

(57) **Abstract**

System for eye tracking during sleep and in altered states of consciousness (1) comprising an eye tracking device including a wearable eye mask (6) comprising an eye mask casing (20) with a form fit border (21) configured for placing over a person's eyes, the eye mask casing (20) and form fit border (21) being opaque configured to prevent light from entering inside of the eye mask casing when worn in a form fit manner by the wearer, an electronic control and communications system (13) and power source (15) for transmission and reception of data with a computing system (2), a humidifier (7) mounted on an inner side of the eye mask casing (20), eye tracking sensors (9) mounted on an inner side of a front panel of the eye mask casing, and an infrared illumination source mounted on an inner side of the eye mask casing and configured to illuminate a position of the eyes of a person wearing the eye tracking device

## Description

### Field of the invention

This invention relates to a system for tracking eye movement during sleep and in altered states of consciousness.

### Background of the invention

The eye and the pupil are easy to access and their dynamics are reliable proxies for various parameters such as overall brain activity, arousal states, level of the autonomic nervous system, cognitive load, sensory processing, dysfunctions of the nervous system, metabolic imbalances, tissue damage in the visual and nervous system. Tracking eye dynamics is thus a cost effective and non-invasive way to get a handle on these biomarkers, which are otherwise measured by electrodes or complex attached sensors.

Eye and pupil tracking are therefore used in many applications ranging from video and virtual reality applications (gaze, attention), transportation and work security (drowsiness detection), ophthalmological (detection of disease), neurological and metabolic diagnostics, in market research or forensics.

Due to the inherent mechanics of the eye, these parameters are only available during the awake state. Having access to pupil and eye dynamics during sleep and altered states of consciousness would allow the tracking of physiological changes much more simply than electrodes, thus enabling simple sleep stage and brain activity monitoring, real time readout of the autonomic nervous system, following disease progression, estimating treatment outcomes and enabling diagnostics.

Pupils and eye movements are hidden behind closed eyelids during natural sleep and altered states of consciousness in humans as the eyelid muscles relax. The above cited parameters are thus not available for monitoring and diagnostics.

Research in animals: mice can naturally sleep with eyes open as a function of sleeping position and pupil and eye dynamics have been described in detail (Yuzgec et al. 2018) and cat eyes have been opened with funnel lenses and sutures which allowed the observation of pupil dynamics (Berlucchi et al. 1964).

Research in humans: mechanical eyelid crutches (Yoss et al. 1970), glue (Rechtschaffen and Foulkes 1965) or tape (Oswald 1960) have been used to keep eyelids artificially open during sleep. Yet this was restricted to short periods and mostly headfixed conditions. In some cases steam from hot water kettles and humidifiers were used to avoid eye damage (Oswald 1960; Rechtschaffen and Foulkes 1965). Recordings are only done for short periods while manually opening the eyelids. Physiological dynamics, disease progression, diagnosis and treatment outcomes often require long term, continuous and repeated measurements. Taken together, eye dynamics were never recorded during extended periods of sleep, due to the lack of comfortable and safe conditions.

Forced and artificial opening of the eyelids with mechanical devices can cause mechanical irritation and discomfort due to corneal drying or cooling, which will lead to rapid awaking. More importantly, the opening of the eyelid during sleep would expose the surface of the eye to potential mechanical damage. Taken together, tracking the pupil size and eye movements during extended periods of sleep (>5 min) is currently an unsolved problem to address.

### Summary of the invention

In view of the foregoing, it is an object of this invention to provide a system for tracking eye parameters during sleep and altered states of consciousness of human subjects for an extended period of time.

It would be advantageous to provide a system for tracking eye parameters that has low discomfort to the person under observation over an extended period of time that may range between one hour and a complete cycles of sleep.

It is advantageous to provide a system for tracking eye parameters during sleep and altered states of consciousness of a human subject that enables the capture of high quality images of pupil size and eye movements in an automated and economical manner.

It is advantageous to provide a system for tracking eye parameters during extended periods of sleep and altered states of consciousness of a human subject that is easy to install, comfortable to wear, rapid to deploy, and simple to operate.

It is advantageous to provide a system for tracking eye parameters during extended periods of that is economical to implement.

Objects of this invention have been achieved by providing a system for tracking eye movement during sleep and in altered states of consciousness according to claim 1.

Dependent claims set forth various advantageous features and embodiments of the invention.

Disclosed herein is a system for eye tracking during sleep and in altered states of consciousness comprising an eye tracking device including a wearable eye mask comprising an eye mask casing with a form fit border configured for placing over a person's eyes, the eye mask casing and form fit border being opaque configured to prevent light from entering inside of the eye mask casing when worn in a form fit manner by the wearer, an electronic control and communications system and power source for transmission and reception of data with a computing system, a humidifier mounted on an inner side of the eye mask casing, eye tracking sensors mounted on an inner side of a front panel of the eye mask casing, and an infrared illumination source mounted on an inner side of the eye mask casing and configured to illuminate a position of the eyes of a person wearing the eye tracking device.

In an advantageous embodiment, the system further comprises environmental sensors including at least a humidity sensor for measuring a humidity level within an inner side of the eye mask casing.

In an advantageous embodiment, the environmental sensors further include a temperature sensor for measuring a temperature on an inner side of the eye mask casing.

In an advantageous embodiment, the eye tracking sensors comprise at least a one pair of cameras, each camera positioned on an inner side of a front panel of the eye mask casing facing an estimated position of a wearer's eye.

In an advantageous embodiment, the humidifier comprises or consists of a water absorbent material mounted on an inside of the eye mask casing.

In an advantageous embodiment, the water absorbent material comprises at least of pair of water absorbent pads mounted on opposite lateral inner sides of the eye mask casing.

In an advantageous embodiment, the electronic control and communications system comprises a circuit board mounted in a housing within the electronic panel of the eye mask casing.

In an advantageous embodiment, the eye tracking device further comprises a thermal management system including a cooling system mounted on a front panel of the eye mask casing for cooling the electronic and control communication system and maintaining a temperature in the eye mask casing at or below a body temperature.

In an advantageous embodiment, the eye tracking device further comprises a display screen mounted on an inner side of a front panel of the eye mask casing configured for displaying an image for visualization by a wearer.

In an advantageous embodiment, the system is provided as a kit including the eye tracking device and eyelid openers in the form of elastic adhesive tapes or another form of elastic mechanism that couples to the eyelids and applies a spring force to spread them apart.

In an advantageous embodiment, the system includes the eye tracking device and a computing system external to the eye tracking device and communicating with the eye tracking device for transfer of data from the eye tracking device in the computing system and for receiving data from the computing system in the eye tracking device for control, feedback, and/or stimulation purposes.

In an advantageous embodiment, the computing system comprises an interface computing unit configured for wireless or wired connection to the eye tracking device for the transfer of images, for instance in the form of a video stream, from the eye tracking device to the interface computing unit, the interface computing unit having program modules installed therein for the processing of the received images to extract eye tracking parameters.

In an advantageous embodiment, the computer system further includes a server having databases installed therein and a machine learning program installed therein for storing eye tracking parameters received from the eye tracking device and for analysing said parameters to output diagnostic and therapeutic results.

In an advantageous embodiment, the eye tracking device further comprises sensory stimulation systems including anyone or more of visual, auditory, olfactory, tactile, thermal, electrical, mechanical stimulation devices.

In an advantageous embodiment, the eye tracking device includes an audio interface system mounted to the eye mask casing, including a microphone for recording sounds generated by the user and speakers for generating sound for the wearer to hear.

Further objects and advantageous aspects of the invention will be apparent from the claims, and from the following detailed description and accompanying figures.

### Brief description of the drawings

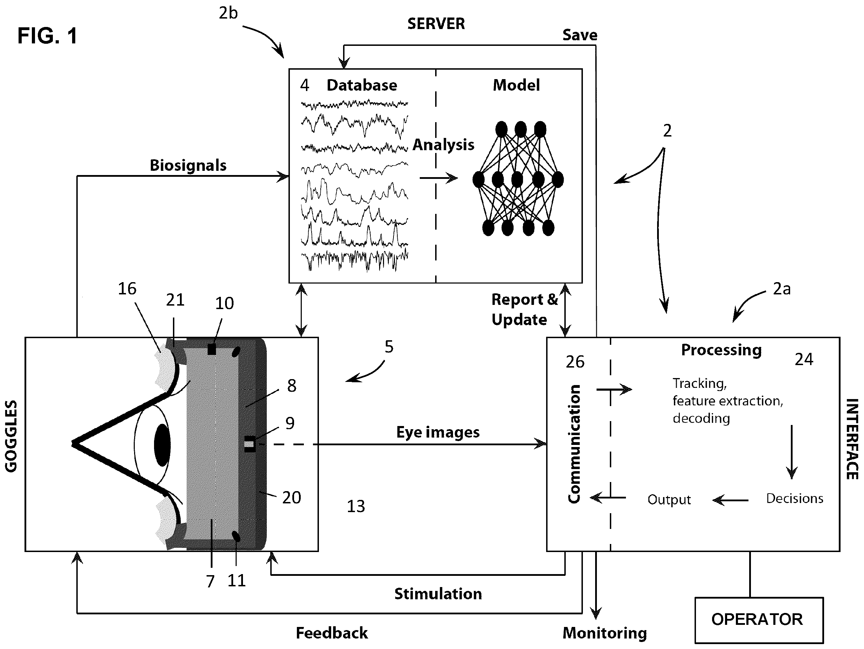

**Figure 1** is a schematic block diagram of a system for tracking eye parameters during extended periods of sleep and altered states of consciousness according to an embodiment of the invention;
**Figure 2a** is a schematic simplified front view of an eye tracking device of the system according to an embodiment of the invention;
**Figure 2b** is an inside view of the eye tracking device of figure 2a;
**Figure 2c** is a schematic illustration of human subject's eye with eyelid openers installed;

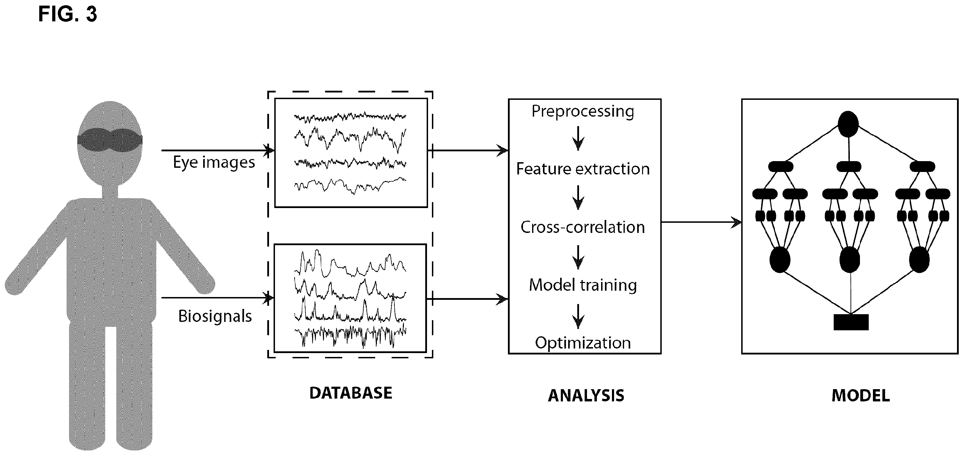

**Figure 3** is a schematic block diagram illustrating a database collection and model generation of a computing system of the system for eye tracking during sleep and altered states of consciousness according to an embodiment of the invention;

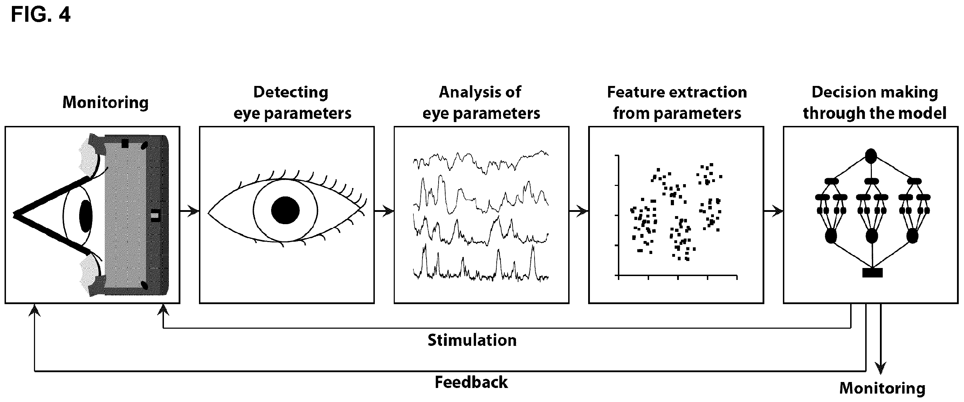

**Figures 4** is a block diagram illustrating a data processing flow while wearing a system for eye tracking during sleep and altered states of consciousness according to an embodiment of the invention;

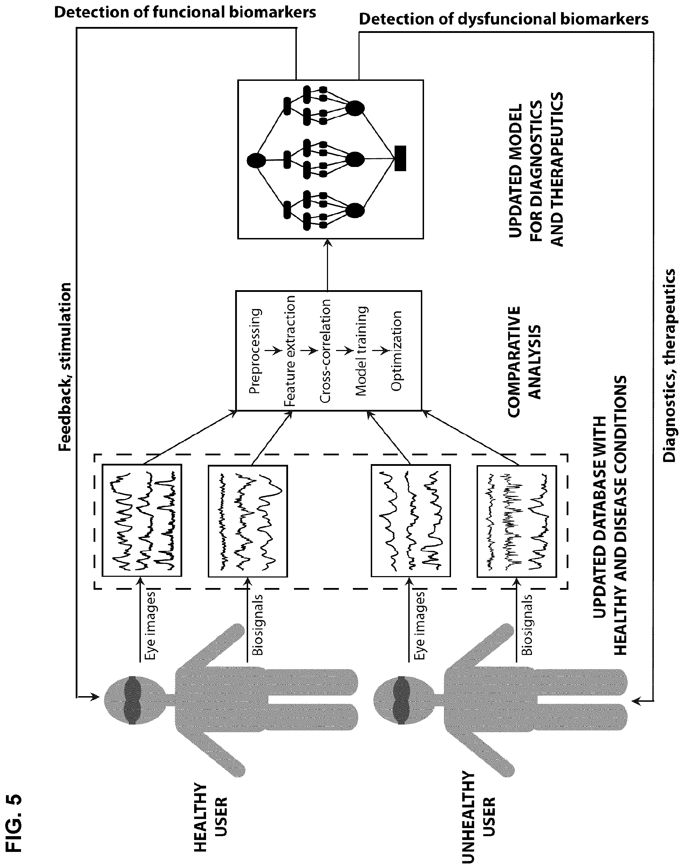

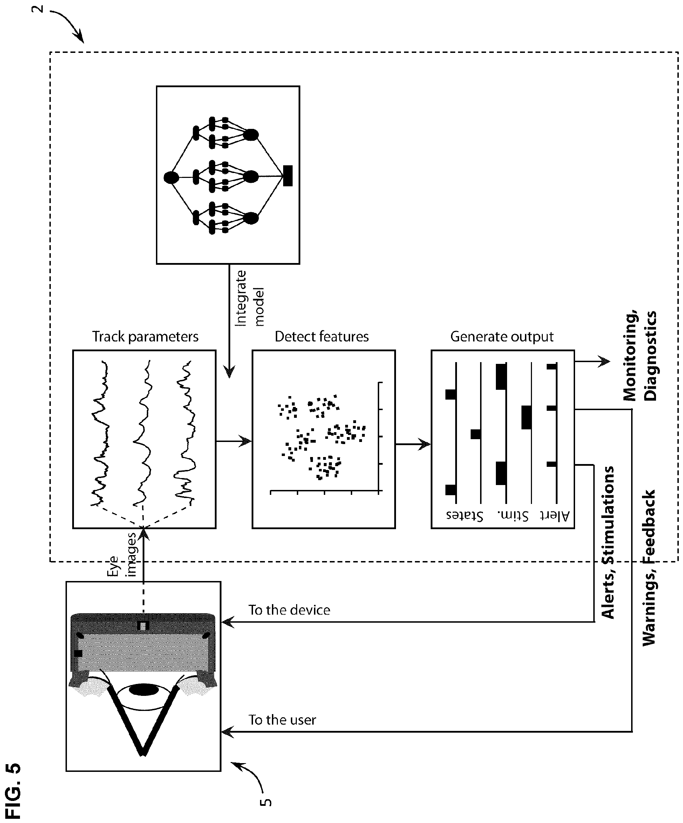

**Figure 5** is a schematic block diagram illustrating a process of diagnostics and therapeutics using a system for eye tracking during sleep and altered states of consciousness according to an embodiment of the invention.

### Detailed description of embodiments of the invention

Referring to the figures, a system for eye tracking during sleep 1 according to embodiments of the invention comprises a computing system 2 and an eye tracking device 5 connected to the computing system 2. The computing system may take various forms and may include various computing units that are connected together in a wired or wireless fashion, in a continuous, intermittent or on demand intercommunication. The computing system may include components such as a server, smartphone, personal computer, or only a single computing unit connected to the eye tracking device 5. Moreover, the eye tracking device 5 may incorporate a portion of the computing system to process data within the eye tracking device prior to transmission to an external computing unit.

For the purposes of example, in the illustrated embodiments, the eye tracking device 5 is connected to an interface unit 2a of the computer system that receives data from the eye tracking device 5 and outputs feedback and other signals to the tracking device, and optionally to other devices such as monitoring systems for diagnostic of therapeutic purposes depending on the application. The interface computing unit may further be connected to a server 2b including databases for storing data or for retrieving data and for performing an analysis of the data using neural networks or other artificial intelligence (machine learning) methods executed by software installed in the server.

The interface unit 2a of the computing system may comprise program modules for processing of data received from the eye tracking device 5, in particular for performing tracking, feature extraction, and decoding of images received from the eye tracking device, for instance in the form of a video stream. The interface unit may however be fully integrated within the eye tracking device 5 which may communicate with a PC or server for transferring processed eye tracking parameters for storage in a database and/or further analysis.

Nevertheless, in a preferred embodiment the interface unit 2a is a separate computing unit that provides an interface for an operator to consult measurements, view eye images and control the eye tracking device remotely and provide stimulation and feedback signals.

The program modules installed in the computing system 3 may include functions for pupil tracking (size, position, rate of displacement) image processing, feature extraction, analysis of the tracking parameters using databases and machine learning algorithms such as neural networks, and program modules for receiving inputs from an operator or external systems.

It may be noted that the system for eye tracking during extended periods of sleep according to embodiments of the invention may be integrated in the eye tracking device or in a medical apparatus or medical system connected to the eye tracking device that measures other parameters such as movement (accelerometer), blood pressure, electrical signals from various organs, and brain activity signals. These other parameters may for instance be correlated with the data received from the eye tracking device along the same timeline for enhanced analysis of various physiological and biological parameters to generate enhanced diagnostic or therapeutic outcomes.

The eye tracking device 5 according to embodiments of the invention may be provided generally in the form of a wearable mask that fits over the subject's pair of eyes, similar in general form for instance to a diver's mask or virtual reality headset, and having for instance an elastic strap to hold the mask against the wearers face over their eyes.

The eye tracking device thus comprises an eye mask 6 with a substantially rigid casing 20 having a form fit border 21 that follows the contour of a person's face around their eyes and over their nose in a generally sealing manner. Some exchange of gas between the inside of the eye mask and outside environment when properly placed on a subject's face is allowed, however the form fit supple border 21 is intended not only to provide a comfortable fit against the subject's face, but also to limit the exchange of gas with the external environment in order to maintain a humid environment and block light from entering the eye mask. The light blocking function of the mask ensures that the inside of the eye mask may be provided with substantially completely darkness to ensure that the subject may sleep while having their eyelids open. The eye mask casing 20 and form fit border 21 are thus opaque and the block substantially all or most of the environmental light from entering within the inside of the eye mask.

The eye tracking device comprises a humidifier 7 which may include an active humidity control system (not shown) or may in certain embodiments simply comprise a water absorbent material, for instance in the form of a water absorbent foam pad positioned within the eye mask casing 20 and that has a sufficient water capacity to provide a relative humidity of close to 100% within the eye mask when positioned on a subject's face during an examination of the patient's sleep, for instance that may be in a range of up to for instance 10 hours, depending on the length of the patient's sleep cycle and the length of the data collection.

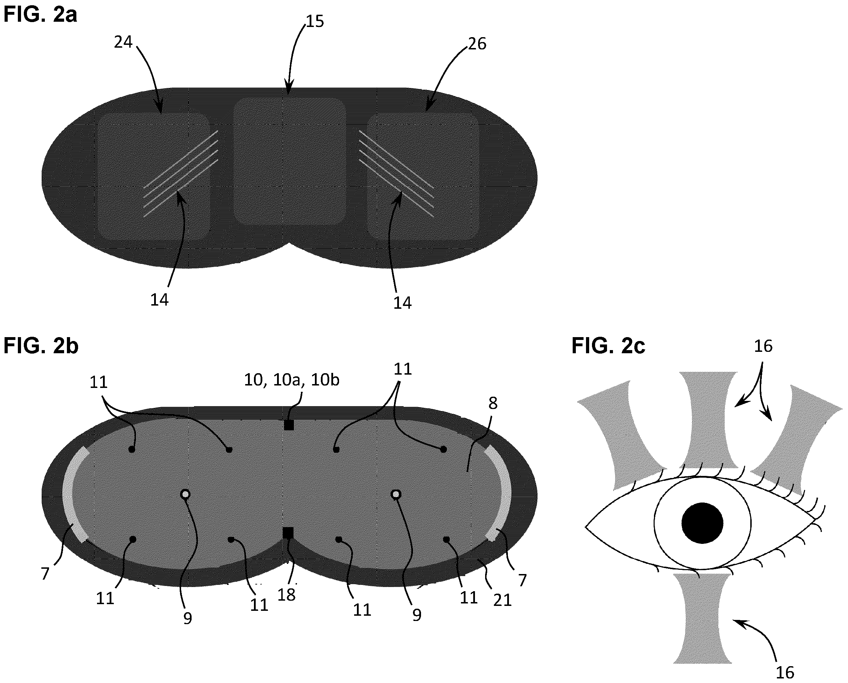

In the illustrated embodiment, the humidifier 7 may for instance comprise a pair of water absorbent pads mounted on an inner lateral side of the eye mask casing schematically illustrated in figure 2b. Water drops may be injected onto the water absorbent material just prior to the installation of the mask on the subject's face before the beginning of the data capture process.

The eye tracking device 5 further comprises eye tracking sensors 9, for instance in the forms of cameras mounted on an inside of a front panel of the casing. There are preferably at least a pair of cameras, one for each of the subject's eyes, for instance positioned within the eye mask casing at a position that is configured to approximately face the pupil of the subject's eye when the eye tracking device is installed for use.

The eye tracking device may further comprise a display screen 8 on an inner side of the eye mask casing front face that may be used to project images. Such images may be projected while the subject is awake, or may be used to project images while the subject is asleep or in an altered state of consciousness in order to determine reactions to visual stimuli during sleep.

The eye tracking device may further include environmental sensors 10 that may in particular include a humidity sensor configured to measure the humidity of the environment on the inside of the eye mask casing. In a passive device, the humidity sensor may simply function to control that the humidity level is sufficiently high during the subject's sleep and in case of a drop in the humidity level, to emit a warning signal. The humidifier may in addition comprise a small container mounted on the of the eye mask casing, for instance connected via a capillary tube to the water absorbent material to provide a reserve of water required for the measurement period.

The environmental sensors may include a temperature sensor to measure the temperature within the eye mask and which may be used to control a thermal management system 14, comprising a cooling system, to maintain the temperature at substantially a body temperature or slightly below. In effect, the electronic control and communication system 13 mounted in the eye tracking device may generate some heat and it may be necessary to evacuate that heat to ensure a controlled temperature inside of the eye mask as mentioned above.

Various cooling systems may be employed, mounted within the casing or on an outer side thereof, for cooling the electronic circuits within the eye mask front panel. Cooling systems are *per se* known, for instance using a Peltier effect or a cooling circuit with a cooling fluid and need not be further described herein.

The eye tracking device 6 further comprises a power source 15, for instance in the form of a rechargeable battery, and an electronic control and communication system 13 including a processor and a communications unit for transfer and reception of data with the external computing unit. Preferably the control and communication system includes a wireless communications module allowing the transfer of data in real time to the interface computing unit 2a, allowing visualization of images by an external operator and remote control of the eye tracking device 5. The remote control may also include sending of stimulation signals from sensory stimulation systems 12 including one or more of audio signals, video signals for display on the display screen 8, electrical signals, or mechanical vibration signals. The eye tracking device may therefore include an audio interface system 18 with a speaker and optionally a microphone. The microphone may be used to capture speech by the subject while awake, and also noise produced by the subject during sleep including breathing noise and speech and other voice noises produced by the subject during their sleep. These audio signals picked up by the microphone during a subject's sleep may thus also be used for diagnostic analysis and therapeutics analysis in conjunction with the eye parameter tracking.

The eye tracking device comprises an illumination source 11 including an infrared illumination source mounted within the eye mask casing, for instance mounted on the inside of the front panel. The infrared illumination is used to capture infrared images by the eye tracking sensor cameras 9 in a frequency spectrum that is not visible by the subject. The provision of complete or substantially complete darkness within the eye mask and humidity control allows the wearer to go to sleep and stay asleep despite having their eyelids open.

The system for eye tracking during the sleep 1 according to embodiments of the invention further comprises a kit that includes the eye tracking device 5 and eyelid openers 16 in the form of elastic tapes with adhesive patches that are stuck to the wearer's eyelids on the one hand and on the wearer's cheek (for the lower eyelid) respectively forehead (for the upper eyelid) on the other hand. When the person's eyelids are closed, the elastic tapes should be in an elastically stretched position. The elastic force of the tapes is configured to open the subject's eyelids when they are asleep and their eyelid muscles are relaxed. The elastic force however allows the eyelids to be closed by the muscular force of the wearer's eyelids, for instance to allow the wearer to blink.

In summary, the system according to embodiments of the invention comprises four important features that work together to allow measurement of eye parameters during extended periods of sleep:
*Eyelid opening system:*
   ➢ Dynamic, soft adhesive tape allows for the opening of the eyelid during sleep while still permitting full closure if the user needs to blink to humidify the eye surface. After the blink the system regains to open position.
*Safe environment:*
   > Eyes are mechanically protected with a covering shield in the shape of an ergonomic mask.
   > The eyes are protected from drying out by a controlled humidification system (including humidity sensors)
   ➢ Eye chamber temperature is controlled to avoid cooling or overheating through temperature sensors
   ➢ Light levels are regulated: complete darkness is needed to sleep with eyes open
*Tracking:*
   ➢ IR light is used to track in full darkness.
   ➢ Miniature infrared cameras provide eye tracking: pupil size, eye movement, coordination parameters
*Sensory stimulation:*
   ➢ Stimulation systems for various sensory modalities (screen: vision, loudspeaker, olfactory or tactile cues) or triggering to stimulate the central and peripheral nerves directly to assess specific biomarkers.
   ➢ Simultaneously tracked parameters and responses to sensory, electrical and other simulations can be used for diagnostics, treatments, monitoring purposes.

The system according to embodiments of the invention allow obtaining pupil/eye movement data for extended time periods in rapid and real-time clinical applications for medical analysis or diagnostics, including:
∘ Detection of brain states
∘ Sleep tracking, sleep disorder diagnosis, disease predisposition and episode detection and related interventions∘ Evaluation of sleep quality and consolidation
∘ Novel nocturnal biomarker
∘ Prediction of learning outcomes as a factor of nocturnal consolidation ∘ Circadian rhythm assessments and treatments
∘ Neurological, neurodegenerative, psychiatric and ophthalmological diagnosis, disease progression monitoring and assessment of treatment outcome
∘ Neurological prognosis, recovery and treatment outcome
∘ Nervous system development, damage, and recovery
∘ Detection, alerting and interruption of disease episodes such as epileptic seizures and apnea
∘ Autonomic nervous system activity, damage and treatment tracking including sympathetic and parasympathetic factors such as breathing, heart rate and sweating
∘ Intracranial pressure diagnosis and monitoring
∘ Anesthetics level tracking
∘ Nociception monitoring, diagnosis and treatment
∘ Monitoring and altering consciousness levels
∘ Neurodegeneration progress monitoring and treatment outcome measurements
∘ Lucid and regular dream detection, monitoring and induction
∘ Nightmare and distress detection in sleep and altered states of consciousness; implementing related interventions
∘ Cognitive enhancement through stimulation and monitoring intervention outcome
∘ Dysfunctions of the autonomic nervous system
∘ Systemic and metabolic disorder diagnosis
∘ Assessments of disease progression and treatment outcomes
∘ Vestibular system assessments and interventions
∘ Closed-loop interventions involving stimulations, treatment and diagnostics
∘ Creating database for function and disease phenotyping

### List of references in the drawings:

**System for eye tracking during sleep 1**
**Computing system 2**
   Interface unit 2a
   Server 2b
   **Program modules / functions 3**
      Pupil tracking
      Feature extraction
      Analysis
      Eye tracking device monitoring
   **Databases 4**
      **Reference**
**Eye tracking device 5**
   **Eye mask 6**
      **Eye mask casing 20**
      **Form fit border 21**
      **Holding strap 22**
   **Humidifier 7**
      Water absorbant material
   **Display Screen 8**
   **Eye tracking sensor(s) 9**
      Camera
   **Environmental sensor(s) 10**
      **Humidity sensor 10a**
      **Temperature sensor 10b**
   **Illumination source 11**
      Infrared illumination source
   **Sensory stimulation systems 12**
      Visual, auditory, olfactory, tactile, thermal, electrical ...
   **Audio interface system 18**
   **Electronic control and communication system 13**
      **Processor 24**
      **Communication interface 26**
   **Thermal management system 14**
      Cooling system
   **Power source 15**
      Battery
   **Eyelid openers 16**
      Elastic adhesive tapes

## Claims

1. A system for eye tracking during sleep and in altered states of consciousness (1) comprising an eye tracking device including a wearable eye mask (6) comprising an eye mask casing (20) with a form fit border (21) configured for placing over a person's eyes, the eye mask casing (20) and form fit border (21) being opaque configured to prevent light from entering inside of the eye mask casing when worn in a form fit manner by the wearer, an electronic control and communications system (13) and power source (15) for transmission and reception of data with a computing system (2), a humidifier (7) mounted on an inner side of the eye mask casing (20), eye tracking sensors (9) mounted on an inner side of a front panel of the eye mask casing, and an infrared illumination source mounted on an inner side of the eye mask casing and configured to illuminate a position of the eyes of a person wearing the eye tracking device.

2. System according to the preceding claim further comprising environmental sensors (10) including at least a humidity sensor for measuring a humidity level within an inner side of the eye mask casing.

3. System according to the preceding claim wherein the environmental sensors further include a temperature sensor for measuring a temperature on an inner side of the eye mask casing.

4. System according to any preceding claim wherein the eye tracking sensors (9) comprise at least a one pair of cameras, each camera positioned on an inner side of a front panel of the eye mask casing facing an estimated position of a wearer's eye.

5. System according to any preceding claim wherein the humidifier (7) comprises or consists of a water absorbent material mounted on an inside of the eye mask casing.

6. System according to the preceding claim wherein the water absorbent material comprises at least of pair of water absorbent pads mounted on opposite lateral inner sides of the eye mask casing.

7. System according to any preceding claim wherein the electronic control and communications system comprises a circuit board mounted in a housing within the electronic panel of the eye mask casing.

8. System according to the preceding claim wherein the eye tracking device further comprises a thermal management system (4) including a cooling system mounted on a front panel of the eye mask casing for cooling the electronic and control communication system and maintaining a temperature in the eye mask casing at or below a body temperature.

9. System according to any preceding claim wherein the eye tracking device (5) further comprises a display screen (8) mounted on an inner side of a front panel of the eye mask casing configured for displaying an image for visualization by a wearer.

10. System according to any preceding claim provided as a kit including the eye tracking device (5) and eyelid openers (16) configured to apply a spring force to open the wearers eyelids during a relaxed muscle state, for instance in the form of elastic adhesive tapes.

11. System according to any preceding claim including the eye tracking device (5) and a computing system (2) external to the eye tracking device and communicating with the eye tracking device for transfer of data from the eye tracking device in the computing system and for receiving data from the computing system in the eye tracking device for control, feedback, and/or stimulation purposes.

12. System according to the preceding claim wherein the computing system comprises an interface computing unit configured for wireless or wired connection to the eye tracking device for the transfer of images, for instance in the form of a video stream, from the eye tracking device to the interface computing unit, the interface computing unit having program modules installed therein for the processing of the received images to extract eye tracking parameters.

13. System according to either of the two directly preceding claims wherein the computer system further includes a server having databases installed therein and a machine learning program installed therein for storing eye tracking parameters received from the eye tracking device and for analysing said parameters to output diagnostic and therapeutic results.

14. System according to any preceding claim wherein the eye tracking device further comprises sensory stimulation systems (12) including anyone or more of visual, auditory, olfactory, tactile, thermal, electrical, magnetic, mechanical stimulation devices.

15. System according to any preceding claim wherein the eye tracking device (5) includes an audio interface system mounted to the eye mask casing (20), including a microphone for recording sounds generated by the user and speakers for generating sound for the wearer to hear.
